(19) **European Patent Office**

Europäisches Patentamt

European Patent Office

Office européen des brevets

(11) **EP 4 702 996 A1**

(12) **EUROPEAN PATENT APPLICATION**

(43) Date of publication:
 04.03.2026 Bulletin 2026/10

(21) Application number: 25197547.0

(22) Date of filing: 22.08.2025

(51) International Patent Classification (IPC):
 **A61L 31/10** (2006.01)

(52) Cooperative Patent Classification (CPC):
 (C-Sets available)
 **A61L 31/10** (Cont.)

(84) Designated Contracting States:
 **AL AT BE BG CH CY CZ DE DK EE ES FI FR GB
 GR HR HU IE IS IT LI LT LU LV MC ME MK MT NL
 NO PL PT RO RS SE SI SK SM TR**
 Designated Extension States:
 **BA**
 Designated Validation States:
 **GE KH LA MA MD TN**

(30) Priority: 29.08.2024 MY PI2024005044

(71) Applicant: **Grow Innovations Sdn Bhd
 42100 Klang Selangor Darul Ehsan (MY)**

(72) Inventors:
 • **Xin, Lim Chia
 42100 Klang (MY)**
 • **Ci, Clara Wong Chia
 42100 Klang (MY)**
 • **Chuan, Lai Fook
 42100 Klang (MY)**
 • **Lui, Ng Kim
 42100 Klang (MY)**
 • **Hoy Kum, Tan Sri Leong
 42100 Klang (MY)**

(74) Representative: **Zacco Denmark A/S
 Arne Jacobsens Allé 15
 2300 Copenhagen S (DK)**

(54) **POWDER FREE ELASTOMERIC ARTICLE HAVING SWEAT ABSORPTION ABILITY AND METHODS OF MAKING THE SAME**

(57) The present invention relates to a powder-free coating composition for elastomeric articles with sweat absorption ability to enhance users' comfortability. The powder-free composition comprises a gelatinized starch dispersion, prepared by combining a source of starch with water under high shearing conditions and subsequently heating it to a temperature between the solubilization and gelatinization temperature of the starch. Additionally, the composition includes thickening agents and preservatives. This innovative formulation eliminates the need for powder-based coatings for sweat absorption while maintaining the desired properties of elastomeric materials, enhancing user comfort, and reducing allergic reactions and environmental contamination associated with traditional powder-coated articles.

EP 4 702 996 A1

(52) Cooperative Patent Classification (CPC): (Cont.)

C-Sets
**A61L 31/10, C08L 3/02**

**Description**

**FIELD OF INVENTION**

The invention relates to a powder free elastomeric article having sweat absorption ability, particularly a powder free elastomeric glove having sweat absorption ability and the method for manufacturing the same.

**BACKGROUND OF THE INVENTION**

Powdered gloves were once popular due to the ease of donning and sweat management provided by the powder, typically cornstarch. However, the use of powdered gloves has largely been phased out because the powder can cause adverse health effects and environmental contamination. These health concerns led the FDA to ban powdered medical gloves in 2017. To address the negative impact of powdered gloves, glove manufacturers are paving the way for the transition from powdered-starch gloves to powder-free gloves. This transition is accompanied by advancements in technology, such as chlorination or acrylic/ polyurethane based polymer coating. For example, US20080034467A1, US8313833B2, WO2006071308, US20220175068A1, US20050132466A1, and US20060141186A1 disclose powderless gloves with an acrylic/polyurethane polymer coating that facilitates easier donning, especially under damp hand conditions. However, these powder-free gloves, produced with polymer coatings, only offer easier donning without providing moisture absorption capabilities, which may lead to issues during use. Users may experience discomfort as the close fit can restrict airflow, causing perspiration and discomfort during prolonged use. In severe cases, this may result in atopic dermatitis, or skin eczema, presenting symptoms like dryness, cracking, swelling, rash, and itching. To address skin eczema arising from perspiration, US20070053958A1 discloses a powderless glove with a coating comprising solubilized rice starch and colloidal oatmeal, which allows the user's skin to be lubricated during wear. However, this powderless coating formed by the solubilized starch does not offer sweat absorption management for users. Additionally, no cost-effective material other than starch has been identified as a coating with good sweat absorption ability to date. Therefore, there is a need to address this gap in the field of elastomeric articles, as conventional methods still rely on starch-based powder for lubrication and moisture absorption.

The aim of the present invention is to transform the powdered starch coating into a thin, powderless coating with stronger sweat-absorbing properties. By applying this powderless starch coating to elastomeric gloves, the invention ensures safer wearing with improved donning and sweat management for end users while ensuring cost-effectiveness during manufacturing process

**SUMMARY OF THE INVENTION**

It is an embodiment of the present invention to provide a powder-free composition for coating an elastomeric article, comprising a starch dispersion thickened by a process further comprising combining a source of starch with water to form a starch dispersion under high shearing conditions; and heating the starch dispersion to a temperature between the solubilization and gelatinization temperature of the starch; thickening agents; and preservatives.

It is an embodiment of the present invention to provide a powder-free composition for coating an elastomeric article wherein the starch source is a natural starch source selected from any one or a combination of corn, potato, tapioca, rice, wheat, barley, arrowroot, or other grains or tubers. The starch source may also be chosen from a modified starch selected from any one or a combination of carboxylated starches, hydroxyethylated starches, resistant starches, thermally oxidized starches, or various types of dextrins. The starch source may also be chosen from a combination of a natural starch and a modified starch.

It is an embodiment of the present invention to provide a powder-free composition for coating an elastomeric article wherein the composition exhibits improved moisture absorption properties due to the process of heating the starch dispersion to a temperature between the solubilization and gelatinization temperature.

It is an embodiment of the present invention to provide a method for manufacturing an elastomeric article with a powder-free coating, comprising the steps of:

- preparing and cleaning a former;

- applying a coagulant to the former;

- dipping the former into an aqueous polymer latex to form a body of the article;

- pre-leaching and subsequent curing of the formed article;

- chlorinating and neutralizing the article;

- post leaching of the article;

- immersing the article into a coating composition for coating an outer surface of the article;

- drying the coated article; and

- removing the article from the former, with outer surface becoming inner coating via inverted stripping;

characterized in that the coating composition is a powder-free coating as described in the above embodiments.

It is an object of the present invention to provide an elastomeric article comprising an outer surface formed from an elastomeric material; an inner surface; and a powder-free coating on the inner surface, said coating comprising a starch dispersion thickened by a process further comprising: combining a source of starch with water to form a starch dispersion under high shearing conditions; and heating the starch dispersion to a temperature between the solubilization and gelatinization temperature of the starch.

**BRIEF DESCRIPTION OF THE DRAWINGS**

Figure 1 illustrates a cross-section of the glove with powder free coating on glove inner surface.
Figure 2 illustrates a cross-section of the glove with powder coating on glove inner surface.
Figure 3 illustrates a flow diagram of a method for manufacturing powderless starch dispersion.
Figure 4 illustrates a flow diagram of glove manufacturing process.

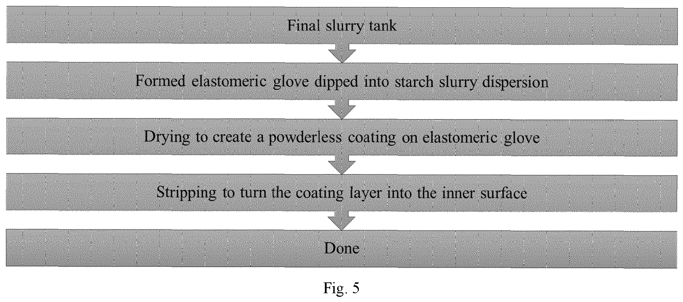

Figure 5 illustrates a flow diagram of powderless sweat-absorbing glove with starch-derived coating.

**DETAILED DESCRIPTION OF THE INVENTION**

It is to be understood that the embodiments disclosed herein are illustrative of the principles of the claims. Other modifications that may be employed are within the scope of the claims. Thus, by way of example, but not of limitation, alternative embodiments may be utilized in accordance with the disclosure herein.

Accordingly, the claims are not limited to embodiments precisely as shown and described. Throughout this specification and the claims, which follow, unless the context requires otherwise, the word "comprise", and variations such as "comprises" or "comprising", will be understood to imply the inclusion of a stated integer or step or group of integers or steps but not the exclusion of any other integer or step or group of integers or steps.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this disclosure relates.

As used herein, the following terms have the following meanings:

"gelatinization temperature" refers to the specific temperature range at which starch granules undergo phase transitions, transforming from a solid state to a gel-like state.

"solubilization temperature" refers to the specific temperature at which a solid substance effectively dissolves in a liquid. At this point, the solid particles break apart and mix uniformly with the liquid.

It is an embodiment of the present invention to provide a coating on an elastomeric article wherein the coating is a powder free composition comprising starch thickened by a novel process of shearing and gelatinization.

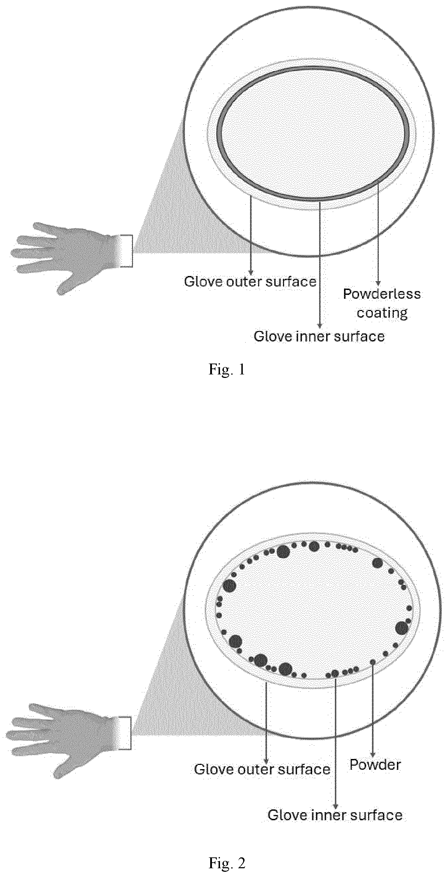

Figure 1 illustrates a glove with powder free coating which comprises an outer surface formed from an elastomeric material selected from but not limited to nitrile rubber, latex or any other elastomeric material and a powder free coating comprising starch thickened via a novel process of shearing and gelatinization. When compared to a conventional glove with powder coating as illustrated by figure 2, the glove illustrated by figure 1 is able to provide a powder free coating solution for thin medical gloves while ensuring effective sweat absorption. It is an object of the present invention to provide a glove which may be but not limited to use as an examination glove, surgical glove, industrial glove or glove for food grade usage.

In a further embodiment of the present invention, it is provided a method for manufacturing an elastomeric article with a powder free coating whereby the powder free coating is manufactured according to the following steps:

(a) a source of starch such as but not limited to potato starch is combined with water (preferably soft water) to form a starch dispersion using a first mixer such as but not limited to a high-speed mixer which provides high shearing conditions preferably at around 6000 rpm. The process of forming a starch dispersion is conducted at room temperature (about 25 °C) for a period of time (preferably about 1 hour).

(b) Upon completion of the mixing process, the starch dispersion should reach a desired temperature which lies between solubilization and gelatinization temperature of starch.

(c) Additional water, thickeners and preservatives are added to the starch dispersion slurry to enhance viscosity and prevent microbial growth during storage.

(d) The starch dispersion slurry is then subjected to overnight mixing using a second mixer at a lower speed such as but not limited to a low-speed mixer which provides low shearing conditions preferably at about 300-500 rpm. The second mixing process is conducted at a constant desired temperature to facilitate gelatinization process of the starch dispersion slurry.

(e) Once the second stirring process has been completed, the starch dispersion slurry is stored at room temperature.

In the processing of the composition for preparation of the powder free coating, the formation of the starch dispersion slurry comprises a natural starch source, thickening agent and preservatives. The starch sources are selected from but not limited to corn, potato, tapioca, rice, wheat, barley, arrowroot and other grains or tubers. Alternatively, the starch can be selected from modified starches which have been modified to enhance their functionalities in industrial applications such as but not limited to carboxylated starches, hydroxyethylated starches, resistant starches, thermally oxidized starches, and various types of dextrins. The starch selected may also be combined and is not limited to a single type of starch. Thickening agents are selected but not limited to gums like guar gum, xanthan gum, locust bean gum, and arabic gum; cellulose derivatives such as methylcellulose, carboxymethyl cellulose (CMC), hydroxyethyl cellulose (HEC), and microcrystalline cellulose and synthetic polymers like polyacrylamide and polyvinyl alcohol (PVA). Preservatives are selected but not limited to phenoxyethanol, chlorphenesin, potassium sorbate and sodium benzoate.

The starch granules of the composition exhibit a semi-crystalline structure with hydrophilic hydroxyl groups, primarily derived from amylose and amylopectin. These hydroxyl groups, composed of oxygen and hydrogen atoms, render amylose and amylopectin highly polar and capable of forming hydrogen bonds with water molecules, contributing to the overall phenomenon of water absorption by starch granules. In addition to the hydrophilic hydroxyl groups, the semi-crystalline structure of starch encompassing both crystalline and amorphous regions, also influences its water absorption properties and powder texture.

In its unheated state, starch granules typically exhibit a higher proportion of crystalline regions compared to amorphous regions. The powdery texture and resistance to swelling associated with unheated starch result from the highly ordered and tightly packed molecular arrangement in these crystalline regions, which restrict the absorption of water molecules.

It is an object of the present invention to prepare a composition that exhibits a higher absorption and a lesser powdery texture after coating onto an elastomeric article. This is because starch undergoes a transformative process known as gelatinization upon heating. In the gelatinization phase, the semi-crystalline structure within starch granules is disrupted. As heat is applied, the crystalline regions start to break down, leading to a notable increase in amorphous regions. This shift to a more disordered molecular arrangement creates a more open structure, allowing water molecules to penetrate and interact more readily, resulting in starch swelling. Consequently, this mechanism leads to improved water absorption by starch, resulting in a gel-like consistency and a loss of the powdery texture associated with the unheated state. In addition to the gelatinization process, the high shear mixing or milling process can further contribute to a reduction in the powdery texture of starch and improve water absorption. The reduction in the powdery texture is achieved by breaking down the starch granule into smaller pieces, whereby the improvement of water absorption is achieved by the release of amylose and amylopectin from the starch granule into the surrounding water.

The composition obtained via the process of high shearing of the starch dispersion between gelatinization and solubilization improves water absorption while reducing the powdery texture of the starch. This process enables the creation of a powder free coating solution for elastomeric articles, ensuring effective moisture absorption.

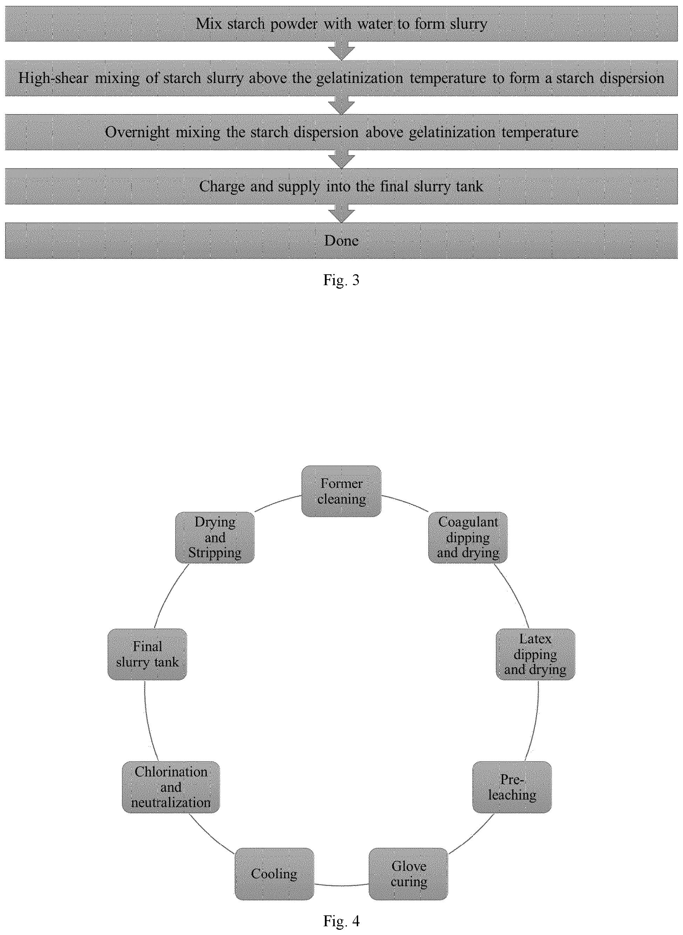

Figure 4 illustrates the process flow of producing an elastomeric article via conventional dipping method incorporating the composition to form a powder free coating on an elastomeric article which is obtained from the processing of starch.

It is therefore an embodiment of the present invention to provide an elastomeric article incorporating a composition for providing a powder free coating on said elastomeric article according to the following steps:

1. A former is prepared and cleaned using acid and alkali wash to remove dirt and residue. A first coat of coagulant typically comprising but not limited to calcium nitrate or carbonate as a latex coagulant and stearate as an antitack agent is applied to the formers to ensure proper glove formation and smooth stripping from said former. (Optionally, wetting agent including Teric 320 can be added to ensure proper former surface wetting for homogenous coagulant coating)

2. The composition is then dipped into aqueous polymer latex which forms the body of the glove. Latex dipping can involve a single or multiple dipping process to achieve the desired thickness.

3. The elastic article formed from the process undergoes pre-leaching to remove excess proteins and impurities, then curing process in an oven to allow warm air to circulate to dry remaining moisture via evaporation and facilitate glove crosslinking process. (After crosslinking, the glove exhibits increased durability, resistance to tearing and stretching, improved chemical and heat resistance, and enhanced structural stability)

4. Once the curing process is completed, the final article is subjected to cooling process, and then undergoes chlorination by dipping the elastomeric article into an aqueous chlorinated solution at 800 - 1200 ppm to improve surface properties. Proper chlorination makes the gloves less tacky and smoother for easy donning.

5. After chlorination, the glove is subjected to neutralization by washing the gloves with a base such as sodium hydroxide or ammonia to remove residual chlorine and adjust the surface pH and post-leaching process to remove remaining impurities.

6. The elastomeric article is then immersed into the starch dispersion slurry formed by the process of figure 3 whereby the outer surface of the glove is coated with the starch dispersion slurry.

7. The elastomeric article then undergoes further drying to allow removal of water which forms a solid coating on the outer surface of the elastomeric article.

8. The coated elastomeric article is then removed from the former via inversion stripping process, whereby the outer surface (while on the former) is inverted to become the inner surface of the elastomeric article as depicted by figure 1. With this production methodology, the final glove exhibits good donning properties facilitated by chlorination and sweat-absorbing ability facilitated by powderless starch coating

It is an object of the present invention to prepare an elastomeric article which can be considered to be thin and powder free. The use of the powder free coating as disclosed herein can create an ultra-thin film on the elastomeric articles (e.g. gloves), resulting in a powderless design. This thin layer of coating absorbs moisture from the skin, reducing the need for traditional powder coatings that can cause allergic reactions or leave residue. The powder free coating also provides a smooth, comfortable feel and enhances the grip properties of the gloves, making them ideal for various applications, especially in healthcare and industrial settings. When assessed according to the ASTM D6124 standard, a mass of less than 2 mg/glove is necessary to be regarded as powder-free gloves. The powderless starch coating when incorporated to the gloves would produce a glove with mass of less than 2 mg thus fulfilling the ASTM standards.

In terms of thickness of the elastomeric article, it is preferred for the glove to be less than 0.2 mm thin, preferably within the range of 0.05 to 0.2 mm in thickness. Gloves which are less than 0.2mm thick are considered to be in the category of thin gloves. Gloves of this thickness are designed to provide enhanced tactile sensitivity, dexterity, and comfort for applications demanding precision and fine motor skills. These gloves are particularly suitable for medical procedures requiring delicate manipulations, such as microsurgery, and industrial tasks involving small or delicate components. The reduced bulk and improved comfort of ultra-thin gloves contribute to enhanced hand-eye coordination, reduced fatigue, and overall improved performance in tasks requiring a high degree of tactile sensitivity and precision. It would therefore be an object of the present invention to prepare an elastomeric article such as a glove which is considered thin gauge gloves which would be less than 0.2 mm in thickness.

**METHODOLOGY**

In the comparative example below, the following equipment was used in the processing of the composition in lab-scale.

1. High Shear Mixer (L5M-A, Silverson, USA) with maximum rotation speed up to 10000 rpm.

2. Overhead stirrer (RW 20, IKA, Germany) with maximum rotation speed up to 2000 rpm.

3. Hotplate stirrer (C-MAG HS 7, IKA, Germany) with speed range 100 - 1500 rpm and heating temperature range 50 - 500 °C.

The particle size of the starch dispersion is examined using Betasizer 2600 to study the swelling of starch granules after water absorption. In characterizing the starch-coated glove, the gloves underwent the ASTM D6124 test to determine the amount of residual powder on them. An in-house testing methodology was also adopted to evaluate the sweat absorption capability of the starch-coated glove. In brief, the film was initially weighed. Subsequently, 0.6 g of a 0.5 % NaCl droplet as simulated sweat was dispersed evenly onto the inner surface of the starch-coated film, followed by a 20-minute absorption process. After 20 minutes, the glove was flipped over on the drying rack and placed under a fume hood with a velocity of 0.5 m/s to remove any unbound excess moisture. The glove was then weighed again after the removal of excess moisture to obtain the final weight. Sweat absorption rate was further compared to the control glove without internal coating using the equation below:

$$\frac{Amount\ of\ Sweat\ Absorbed\ for\ Starch\ of coated\ glove - Amount\ of\ Sweat\ Absorbed\ for\ Control\ Glove}{Amount\ of\ Sweat\ Absorbed\ for\ Control\ Glove}\ x\ 100\%$$

Example 1A

Potato starch is combined with soft water to form a starch dispersion using a high-speed mixer (L5M-A) under high-shearing conditions at 6000 rpm, and the process was conducted at room temperature (~25 °C) for 1 hour. The temperature of the starch dispersion was about 65 °C after 6000 rpm mixing, which is between the solubilization and gelatinization temperature of the potato starch. Additional water, thickener, and preservatives were added to the concentrated starch slurry, with the final composition detailed in Table 1. After this, the starch slurry was subjected to overnight mixing at low speed (300-500 rpm) using a hotplate stirrer (C-MAG HS 7), maintaining a temperature of approximately 65 °C to facilitate the gelatinization process under storage conditions for a minimum of 16 hours. After the overnight storage, the result was a stable starch dispersion that does not form powder sedimentation at room temperature.

Example 1B

The starch slurry of 1B underwent a similar process and formulation, differing only in the mixing methods. Mixing was conducted using an overhead mixer (RW 20) at low-speed conditions of 1000 rpm. Subsequently, overnight mixing was performed using a hotplate stirrer (C-MAG HS 7) at low speed (300-500 rpm), but under room temperature conditions (approximately 25 °C). This mixing process is the conventional method for preparing starch slurry for powdered gloves, occurring well below the gelatinization temperature. Following overnight storage, the result was an unstable starch dispersion that exhibited powder sedimentation at room temperature.

Example 1C

The starch slurry underwent a similar process and formulation, with the exception that mixing was carried out using a high-speed mixer (L5M-A) under extremely high-shearing conditions at 9000 rpm. The temperature of the starch dispersion rose to 80 °C after the 9000-rpm mixing, surpassing the solubilization temperature of the potato starch. Overnight mixing was also conducted at 80 °C, 300-500 rpm, using a hotplate stirrer (C-MAG HS 7). After overnight storage, the result was a transparent starch dispersion, indicating complete solubilization of potato starch at room temperature.

Table 1: Composition of starch dispersion and synthesis parameters.

|  |  | 1A | 1B | 1C |
|---|---|---|---|---|
| Raw material | Potato starch (%) | 1 | 1 | 1 |
|  | Thickener (%) | 0.25 | 0.25 | 0.25 |
|  | Preservative (%) | 0.1 | 0.1 | 0.1 |
|  | Soft water (%) | 98.65 | 98.65 | 98.65 |
| Mixing speed (RPM) | | 6000 | 500 | 8000 |
| Temperature after mixing (°C) | | 65 | 25 | 80 |
| Temperature during overnight storage (°C) | | 65 | 25 | 80 |

The original particle size (D90) of the potato starch dispersion was 68.6 $\mu$m. When the starch dispersion synthesis was carried out above the gelatinization temperature (Example 1A), the particle size of the potato starch dispersion increased to 243.7 $\mu$m. This indicated significant water absorption, resulting in a 355 % increase in starch swelling after synthesis as shown in Table 2. For comparison, when the starch dispersion synthesis was carried out below the gelatinization temperature (1B), a lower swelling extent of 250 % was observed. However, when the starch dispersion synthesis was carried out under extremely high temperatures (1C), the starch granules burst, with the majority of components derived from amylose and amylopectin solubilizing into the aqueous liquid environment, rendering the particle size undetectable.

The synthesized starch dispersion was applied onto the glove using the conventional glove dipping process, and the sweat-absorption of the starch-coated glove was compared to a control glove without any coating (Table 2). For sweat absorption testing, the gloves underwent a 20-minute absorption process, mirroring the average glove-changing period for healthcare workers. The control glove absorbed 40 mg of simulated sweat after a 20-minute absorption process. In comparison, the glove coated with gelatinized starch dispersion (1A) exhibited the highest sweat absorption at 120 mg, which was 300 % higher than the control's sweat absorption. This was followed by the glove coated with starch slurry prepared by conventional method (1B), which absorbed 66 mg of moisture, demonstrating a 66 % improvement in sweat absorption compared to the control. Intriguingly, the glove coated with solubilized starch dispersion (1C) showed no sweat absorption (-100%) compared to the control glove. These findings highlight the novelty of the starch dispersion synthesis for the glove coating, which further improves the moisture-absorption rate of the starch-coated glove compared to the powdered glove prepared via conventional starch slurry preparation.

Regarding glove powder content, the control glove exhibited an insignificant amount of powder, with only 0.04 mg/pc of the glove as demonstrated by Table 2. This is well below the ASTM D6124 requirements for a powderless glove, which specify a maximum of 2 milligrams of total powder particulate on the finished glove. Conversely, gloves treated with gelatinized starch dispersion (1A) and solubilized starch dispersion (1C) exhibited powder contents of 1.00 mg/pc and 0.42 mg/pc of the glove, respectively, both falling below the standard requirement of 2 mg/pc. Upon usage, the gloves produced from process 1A and 1C did not leave any powder residue on the hand, but a significant amount of powder residue was observed for 1B after removing the glove. These findings underscore the novelty of starch dispersion synthesis for glove coating, which effectively minimizes the powdery texture of the starch-coated glove compared to the powdered glove prepared via conventional starch slurry preparation.

Table 2. The percentage of particle swelling in the starch dispersion, along with percentage differences in sweat-absorption and glove powder content compared to control glove.

| | Swelling % of the dispersion | Differences in sweat-absorption compared to control glove (%) | Glove powder content (mg/pc) |
|---|---|---|---|
| Control glove (Uncoating) | N/A | | 0.04 |
| 1A | 355 | 276.2 | 1.00 |
| 1B | 250 | 65.7 | 3.86 |
| 1C | No particle size | -100 | 0.42 |

In comparison, example 1B depicts starch granules exhibiting a higher proportion of crystalline regions compared to amorphous regions due to it being in an unheated state. This causes a powdery texture on the glove that exceeds the maximum of 2 milligrams of total powder particulate, making it resistant to swelling and restricting the absorption of water molecules. The starch dispersion synthesized in example 1A on the other hand exhibits a high absorption of water molecules and no powdery texture upon coating on a glove. This is due to the starch granules undergoing gelatinization upon heating. In gelatinization phase, the semi-crystalline structure within starch granules is disrupted. As heat is applied, the crystalline regions start to break down, leading to a notable increase in amorphous regions. This shift to a more disordered molecular arrangement creates a more open structure, allowing water molecules to penetrate and interact more readily, resulting in starch swelling. Consequently, this mechanism leads to improved water absorption by starch, resulting in a gel-like consistency and a loss of the powdery texture associated with the unheated state. In addition to the gelatinization process, the high shear mixing or milling process can further contribute to a reduction in the powdery texture of starch and improve water absorption. The reduction in the powdery texture is achieved by breaking down the starch granule into smaller pieces, whereby the improvement of water absorption is achieved by the release of amylose and amylopectin from the starch granule into the surrounding water.

Example 1C on the other hand involves overheating or shearing of the starch. This induced solubilization, a process characterized by the bursting of starch granules with no remaining crystallinity. The solubilized starch formed a uniformly dispersed mixture at the molecular level in and with the aqueous liquid, rendering the particle size undetectable. Following solubilization, the subsequent drying of the starch dispersion could lead to recrystallization, whereby starch molecules reorganized, forming a more ordered and crystalline structure. The starch's molecular configuration became less amenable to interacting with water molecules, resulting in a film with minimal water absorption capacity. The combination of overheating-induced solubilization followed by recrystallization during drying led to a starch film with reduced water absorption properties of the composition.

**Claims**

1. A powder-free composition for coating an elastomeric article, comprising a starch dispersion formed by a process of combining starch with water under high shearing conditions and heating the combination to a temperature between the solubilization and gelatinization temperature of the starch.

2. A powder-free composition for coating an elastomeric article comprising a starch dispersion formed according to claim 1, thickening agents and preservatives.

3. The powder-free composition according to any one of claims 1 or 2, wherein the starch is a natural starch source selected from any one or a combination of corn, potato, tapioca, rice, wheat, barley, arrowroot, or other grains or tubers.

4. The powder-free composition according to any one of claims 1 or 2, wherein the starch is a modified starch selected from any one or a combination of carboxylated starches, hydroxyethylated starches, resistant starches, thermally oxidized starches, or various types of dextrins.

5. The powder-free composition according to any one of claims 1 to 2, comprising a combination of a natural starch and a modified starch.

6. The powder-free composition according to any one of claims 1 to 5, wherein the thickening agents are selected from gums such as guar gum, xanthan gum, locust bean gum, and arabic gum; cellulose derivatives such as methylcellulose, carboxymethyl cellulose (CMC), hydroxyethyl cellulose (HEC), and microcrystalline cellulose and synthetic polymers like polyacrylamide and polyvinyl alcohol (PVA).

7. The powder-free composition according to any one of claims 1 to 6, wherein the preservatives are selected from phenoxyethanol, chlorphenesin, potassium sorbate and sodium benzoate.

8. The powder-free composition according to any one of claims 1 to 7, wherein the composition exhibits improved moisture absorption properties due to the process of heating the starch dispersion to a temperature between the solubilization and gelatinization temperature.

9. A method for manufacturing an elastomeric article with a powder-free coating, comprising the steps of:

    preparing and cleaning a former;
    applying a coagulant to the former;
    dipping the former into an aqueous polymer latex to form a body of the article;
    pre-leaching and subsequent curing of the formed article;
    chlorinating and neutralizing the article;
    post leaching of the article;
    immersing the article into a coating composition for coating an outer surface of the article;
    drying the coated article; and
    removing the article from the former, with outer surface becoming inner coating via inverted stripping;
    **characterized in that** the coating composition is a powder-free coating according to any one of claims 1-8.

10. The method of claim 9, wherein the coagulant is selected from the group consisting of calcium nitrate, calcium carbonate, and calcium stearate.

11. An elastomeric article comprising:

    an outer surface formed from an elastomeric material;
    an inner surface; and
    **characterized in that** a powder-free coating is applied on the inner surface, said coating comprising a starch dispersion formed by a process of combining starch with water under high shearing conditions and heating the combination to a temperature between the solubilization and gelatinization temperature of the starch.

12. The powder-free composition of any one of claims 1-8, method of claim 9 and elastomeric article of claim 11, wherein the elastomeric material is selected from nitrile rubber or latex.

13. The powder-free composition of any one of claims 1-8, method of claim 9 and elastomeric article of claim 11-12, wherein the elastomeric material is a glove.

Fig. 1

Fig. 2

Mix starch powder with water to form slurry

High-shear mixing of starch slurry above the gelatinization temperature to form a starch dispersion

Overnight mixing the starch dispersion above gelatinization temperature

Charge and supply into the final slurry tank

Done

Fig. 3

Former cleaning

Coagulant dipping and drying

Drying and Stripping

Latex dipping and drying

Final slurry tank

Chlorination and neutralization

Pre-leaching

Cooling

Glove curing

Fig. 4

Final slurry tank

↓

Formed elastomeric glove dipped into starch slurry dispersion

↓

Drying to create a powderless coating on elastomeric glove

↓

Stripping to turn the coating layer into the inner surface

↓

Done

Fig. 5

Europäisches
Patentamt
European
Patent Office
Office européen
des brevets

## EUROPEAN SEARCH REPORT

**Application Number**

EP 25 19 7547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2007/053958 A1 (NEUSER JOSEPH H [US] ET AL) 8 March 2007 (2007-03-08)<br>* claims 1,2,4,11,15 *<br>----- | 1-13 | INV.<br>A61L31/10 |
| A,P | WO 2024/219958 A1 (HARTALEGA RES SDN BHD [MY]) 24 October 2024 (2024-10-24)<br>* claims 1,4,6,19 *<br>* page 9, line 3 - page 10, line 5 *<br>* page 14, lines 6-11 *<br>----- | 1-13 | |
| | | | TECHNICAL FIELDS SEARCHED (IPC)<br><br>A61L |

The present search report has been drawn up for all claims

| Place of search | Date of completion of the search | Examiner |
|---|---|---|
| The Hague | 6 January 2026 | Zalfen, Alina |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 19 7547

This annex lists the patent family members relating to the patent documents cited in the above-mentioned European search report.
The members are as contained in the European Patent Office EDP file on
The European Patent Office is in no way liable for these particulars which are merely given for the purpose of information.

06-01-2026

| Patent document cited in search report | Publication date | Patent family member(s) | | Publication date |
|---|---|---|---|---|
| US 2007053958 A1 | 08-03-2007 | US | 2007053958 A1 | 08-03-2007 |
| | | US | 2010229281 A1 | 16-09-2010 |
| | | US | 2012102620 A1 | 03-05-2012 |
| WO 2024219958 A1 | 24-10-2024 | AU | 2023443961 A1 | 12-06-2025 |
| | | CN | 121002134 A | 21-11-2025 |
| | | EP | 4612245 A1 | 10-09-2025 |
| | | MY | 208456 A | 09-05-2025 |
| | | WO | 2024219958 A1 | 24-10-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080034467 A1
- US 8313833 B2
- WO 2006071308 A
- US 20220175068 A1
- US 20050132466 A1
- US 20060141186 A1
- US 20070053958 A1